# EUROPEAN PATENT SPECIFICATION

(11) **EP 2 897 955 B1**
(45) Date of publication and mention of the grant of the patent: **06.11.2019**
(21) Application number: 13836301.5
(22) Date of filing: 10.09.2013
(51) Int. Cl.: C07D 451/10, A61K 31/46, A61P 11/06

(54) **NEW TIOTROPIUM BROMIDE CRYSTALLINE FORM**
NEUE KRISTALLINE FORM VON TIOTROPIUMBROMID
NOUVELLE FORME CRISTALLINE DU BROMURE DE TIOTROPIUM

(30) Priority: 11.09.2012 TR 201210358
(43) Date of publication of application: 29.07.2015
(73) Proprietor: Bilgic, Mahmut, 34220 Esenler-Istanbul (TR)
(72) Inventor: BLATTER, Fritz, CH-4303 Kaiseraugst (CH); RÖDEL, Eva, CH-4303 Kaiseraugst (CH)
(86) International application number: PCT/TR2013/000290
(87) International publication number: WO 2014/042605

(56) References cited:
- WO-A1-03/000265
- WO-A1-03/078429
- WO-A1-2005/042527
- WO-A1-2012/026906
- WO-A1-2012/118461
- WO-A1-2012/118462
- WO-A1-2013/117886
- WO-A1-2013/143510
- WO-A2-2006/117299
- WO-A2-2006/117300
- WO-A2-2007/075858
- WO-A2-2011/015882
- CN-A- 1 634 921
- CN-A- 103 130 798
- US-A1- 2003 087 927
- US-A1- 2003 185 766
- US-A1- 2005 131 007
- US-A1- 2005 143 410
- "Crystalline (1[alpha],2[beta],4[beta],5[alpha],7[beta] )-7-[(hydroxydi-2-thienylacetyl)oxy]-9,9-d imethyl-3-oxa-9-azoniatricyclo[3.3.1.0]non ane bromide and process for preparation thereof", IP.COM JOURNAL, IP.COM INC., WEST HENRIETTA, NY, US, 30 November 2006 (2006-11-30), XP013116939, ISSN: 1533-0001

## Description

The present invention is related to a method for producing a crystalline compound comprising Tiotropium bromide.

Tiotropium bromide disclosed in EP 0 418 716 A1 has the following chemical structure:

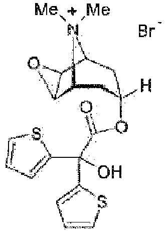

Tiotropium bromide is a highly effective anticholinergic with a long lasting effect. It can be used for treating respiratory complaints and particularly COPD (chronic obstructive pulmonary disease) and asthma.

For treating the abovementioned complaints, Tiotropium bromide is preferably administered by the inhalation route. In addition to the administration of broncholytically active compounds in the form of metered aerosols and inhalable solutions, use of inhalable powders containing active substance is of particular importance.

Tiotropium bromide is preferably administered by the inhalation route. Suitable inhalable powders packed into appropriate capsules or blisters may be used. Alternatively, it may be administered by the use of suitable inhalable aerosols. These also include powdered inhalable aerosols which contain, for example, HFA134a, HFA227 or mixtures thereof as propellant gas.

Since Tiotropium bromide is very potent, only a small amount of the pharmaceutically active substance suffices per single dose to achieve the desired therapeutic effect. Thus, the pharmaceutically active substance has to be diluted in a suitable excipient in order to prepare an inhalable powder. The amount of inhalable composition must be precisely metered in every single dose. Because of the large amount of excipient, the properties of the inhalable powder are significantly influenced by the choice of the excipient. Moreover, the particle size of the pharmaccutically active substance and of the excipient is very important for the emptying characteristics of capsules or blisters when used in an inhaler.

The active substance particles that shall be administered by the inhalation route should optimally meet essential requirements such as appropriate aerodynamic particle size, appropriate particle shape, uniformity of particle size distribution, low aerodynamic dispersion forces, low density, high physical and chemical stability.

Additionally, other physical properties of the pharmaceutically active substance such as the absorption of water and the ease of grinding of the pharmaceutically active substance are of great importance.

The correct manufacture of the abovementioned compositions which are suitable for use for the administration of a pharmaceutically active substance by the inhalation route is based on various parameters which are connected with the nature of the active substance itself. In pharmaceutical compositions which are used like tiotropium bromide in the form of inhalable powders or inhalable aerosols, the crystalline active substance is used in ground (micronized) form for preparing the formulation. Since the pharmaceutical quality of a pharmaceutical formulation requires that the pharmaceutically active substance should always have the same crystalline modification, the stability and properties of the crystalline active substance are subject to stringent requirements from this point of view as well.

Furthermore, micronized powders to be taken by the inhalation route which have moisture ratio as low as possible prevent agglomeration of the product and provide it to be transmitted into the target area more effectively.

One of the many crystal forms of anhydrous tiotropium bromide disclosed in the application numbered WO 2012/118462 with the following characterization parameters. Monoclinic elementary cell parameters are; a=18.82 Å b=11.38 Å c=11.71Å β=120.96°. This crystal form of tiotropium bromide disclosed as suitable to prepare useful tiotropium bromide formulations. The applicant has carried out studies in this regard to produce a crystal form with better properties by a more advantageous method.

Therefore, one objective of the present invention is to develop a method for producing anhydrous tiotropium bromide which is suitable to produce more stable compounds comprising tiotropium bromide.

It is an objective of the present invention to provide Tiotropium bromide in salt form having good chemical and/or physical stability and/or good processability during both its preparation as a pharmaceutically active substance and in the preparation of pharmaceutical compositions containing Tiotropium bromide.

It is a further objective of the invention to provide crystalline salts of Tiotropium bromide that are accessible in a reproducible manner and constant quality in inexpensive and well controlled production processes.

The fact that tiotropium bromide has therapeutic efficacy as a pharmaceutically active substance even at very low doses indicates the need in the technical field to find other effective substances for administration in inhalable composition form. Thus, a further technical problem that the present invention relates to is to prepare practical dosage forms of Tiotropium bromide in inhalable composition form.

In another aspect, the present invention is related to production of stable tiotropium bromide salts which are obtained repeatably by an appropriate method.

While the crystalline forms in the prior art give a single crystalline form in batches of small amounts, variations are seen in the crystalline form as amounts of batches increase and mixtures of crystalline forms are obtained. This poses significant problems for manufacturers and prevents manufacture of the product in high amounts and of high quality. The technical problem underlying the present invention has been solved by a method for producing the compound (Tiotropium bromide) figured in Formula I and having monoclinic elementary cell parameters of a = 9.94 Å, b =11.11.95 Å, c = 18.57 Å, β = 108.47°.

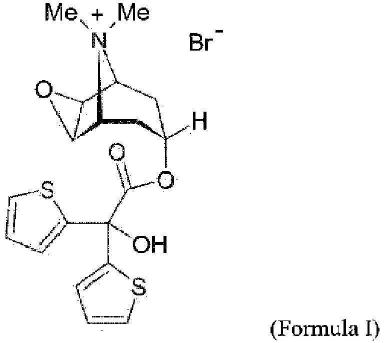

The method of the present invention produces a crystalline compound that can be mixed with suitable excipients so as to obtain an inhalable composition. The crystalline compound can be ground in order to adjust particle size precisely.

Grinding process can be applied to the crystalline compound produced by the method of the present invention singly as well as applied to the crystalline compound produced by the method of the present invention which is in combination with an excipient. At this point, grinding process can be performed by using the methods of hammer mill, jet mill, blade mill etc. Grinding process can be performed before preparation of the pharmaceutical composition comprising the crystalline compound produced by the method of the present invention as well as during preparation of the pharmaceutical composition or before post-production storage of the pharmaceutical composition prepared.

In the case that blade mill is used, pulverization process is performed by the impact of the rotating blades in the device.

In the case that hammer mill is used, pulverization is performed by the impact of the rotating hammers in the device.

In the case that jet mill is used, pulverization is performed by providing collision of the particles with each other with the help of high-pressured and high-speed airstream.

Furthermore, the inhalable composition comprising the crystalline compound produced by the method of the present invention can be stored without losing its effectiveness.

In addition to this, the crystalline compounds produced by the method of the present invention are also stable in accelerated conditions. The crystalline compound can be stored at 40 °C without presenting any degradation or transformation. Consequently, the crystalline compound of the present invention can be used as a drug also in the countries which generally have high temperatures. The drug does not lose its effectiveness under high temperature.

In addition, crystalline compounds produced by the method according to the present invention are stable under accelerated conditions. It is possible to store the crystalline compound at a temperature of 40 °C without any decomposition or conversion. Consequently, the crystalline compound produced by the method of the present invention can be used as medicament in countries generally having high temperatures. At elevated temperature, the medicament will not lose effectiveness.

The inventors have found that the crystalline form produced by the method of the present invention can be obtained purely, without any degradation in the crystalline form of the final product and/or without forming any other crystalline form in the final product both in small amounts and high amounts by the same process. In this regard, the crystals produced by the method of the present invention have the advantages of enabling production without any chemical or polymorphic impurity in high amounts in addition to the superior chemical and physical stability they possess.

Pharmaceutical compositions may comprise a pharmaceutically acceptable, nontoxic and therapeutically effective amount of the crystalline form of tiotropium bromide produced by the method of the present invention. The pharmaceutical compositions comprising the crystalline form of tiotropium bromide produced by the method of the present invention are in the form of dry powder or pressurized metered dose-inhalation composition, preferably dry powder inhalation composition.

During preparation process of dry powder inhalation compositions, two methods are commonly implemented in order to transmit effective amount of the drug to the targeted area. One of them is based on controlled agglomeration of undiluted drug; the other one is based on adhesion of micronized drug particles to the surface of an inert carrier having large particle size. The pharmaceutical compositions comprising the crystalline form of tiotropium bromide produced by the method of the present invention are preferably prepared by implementing the second method. When the second method is implemented, the pharmaceutical composition comprises at least one pharmaceutically acceptable inert carrier and optionally at least one phannaccutically acceptable excipient different from the carrier(s) along with the active agent.

The term "micronized drug particles" refers to the crystalline form of tiotropium bromide produced by the method in accordance with the present invention.

Tiotropium bromide in crystalline form produced by the method in accordance with the present invention is characterized by having an average particle size in the range of 1 - 10µm, preferably in the range of 1-5 µm. The pharmaceutical compositions comprising the crystalline form of tiotropium bromide produced by the method of the present invention are characterized by comprising crystalline form of tiotropium bromide produced by the method of the present invention in the range of 0.001-50%, preferably in the range of 0.01-10%.

The term "inert carrier" signifies that the carrier can be selected from a group comprising monosaccharides such as glucose or arabinose; disaccharides such as lactose, saccharose, maltose; polysaccharides such as oligosaccharides, dextrans; polyalcohols such as sorbitol, mannitol, xylitol; salts such as sodium chloride, calcium carbonate. Preferably lactose, more preferably lactose monohydrate or anhydrous lactose is used.

The pharmaceutical compositions comprising the crystalline form of tiotropium bromide produced by the method of the present invention can comprise at least one inert carrier having large particle size or small particle size and optionally at least one excipient together. The inert carrier having large particle size is characterized by having an average particle size (d₅₀) in the range of 10-250 µm, preferably in the range of 10-150 µm, more preferably of 150 µm; the inert carrier having small particle size, on the other hand, is characterized by having an average particle size (d₅₀) in the range of 1-10 µm, preferably of 10 µm. The inert carriers having large particle size and small particle size can be the same or different compounds.

Depending on formulation strategy, at least one pharmaceutically acceptable excipient can be selected from propellant gases (propellants) such as chlorofluorocarbons, hydrofluoroalkanes and hydrocarbons; surface active agents (surfactants) such as oleic acid, polysorbates, propylene glycol, polyethylene glycol, cetyl alcohol, stearyl alcohol, sorbitan fatty acid esters, sugar esters of fatty acids, glycerides of fatty acids, isopropyl myristate and lecithin; cosolvents such as ethanol, water and diethyl ether; antioxidants such as butylated hydroxyanisole (BHA), sodium ascorbate, butylated hydroxytoluene (BHT), sodium sulphide, gallats (such as propyl gallate), tocopherol, citric acid, malic acid, ascorbic acid, acetylcysteine, fumaric acid, lecithin, ascorbyl palmitate, ethylenediamine tetraacetate; carbohydrates such as sweeteners, lactose, glucose, fructose, galactose, sucrose, maltose, trehalose, maltodextrins, dextrans, cyclodextrins, starch and cellulose; polyalcohols such as sorbitol, mannitol and xylitol; aminoacids such as glycine, arginine, lysine, aspartic acid and glutamic acid; peptides such as human serum albumin; gelatine; various salts and taste masking agents. The said at least one excipient is not limited to these substances.

In preparation process of pressurized metered-dose inhalation compositions, two formulation strategies are implemented depending on physicochemical characteristics of the active agent and propellant gas system. One of them is solution, the other one is suspension formulation. The pharmaceutical composition preferred comprises propellant gases, surfactants and at least one basic excipient selected from the group of co-solvents and optionally at least one other pharmaceutically acceptable excipient along with the active agent.

The term "active agent" refers to the crystalline form of tiotropium bromide produced by the method of the present invention. The crystal of tiotropium bromide produced by the method of the present invention is characterized by having an average particle size in the range of 1-10µm, preferably in the range of 1-5µm. The pharmaceutical compositions comprising the crystalline form of tiotropium bromide produced by the method of the present invention are characterized by comprising the crystalline form of tiotropium bromide produced by the method according to the present invention in the range of 0,001-50,000%, preferably in the range of 0.01-10.00%.

The term "average particle size" refers to 50% of volume distribution measured with a laser diffractometer. According to this, 50% of the particles has a smaller particle size than the given particle size by volume, 50% of the particles has a larger particle size than the given particle size by volume.

The pharmaceutical compositions comprising the crystalline form of tiotropium bromide produced by the method of the present invention can additionally comprise at least one active agent selected from drugs such as other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors. The crystalline form of tiotropium bromide produced by the method of the present invention can be used separately, sequentially or simultaneously with at least one active agent selected from the specified group.

Another characteristic feature of the present invention is that the pharmaceutical compositions comprising the crystalline form of tiotropium bromide produced by the method of the present invention are used in treatment of respiratory tract diseases, particularly asthma and COPD (chronic obstructive pulmonary disease).

Preparation methods of the pharmaceutical compositions comprising the crystalline form produced by the method of the present invention

Preparation process of dry powder inhalation compositions comprising the crystalline form of tiotropium bromide produced by the method of the present invention comprises the following steps:
- sieving inert carriers having large particle size and small particle size separately, obtaining the first mixture as a result of adding the sieved inert carrier having small particle size into tiotropium bromide in crystalline form produced by the method in accordance with the present invention,
- sieving the first mixture obtained,
- obtaining the second mixture as a result of adding the inert carrier sieved having large particle size into the first mixture,
- sieving and mixing the second mixture obtained,
- making the final mixture ready to be filled into capsules/blisters.

Preparation process of the pressurized metered-dose inhalation compositions comprises the following steps:
- cooling the production vessel to -25°C,
- pumping propellant gas into the vessel,
- adding tiotropium bromide in crystalline form produced by the method in accordance with the present invention into the vessel and mixing the mixture obtained,
- filling the final mixture into the appropriate containers.

The terms "crystalline form of tiotropium bromide of the present invention" or "tiotropium bromide in crystalline form in accordance with the present invention" refer to tiotropium bromide having monoclinic elementary cell parameters of a = 9.94 Å, b =11.95 Å, c = 18.57 Å, β = 108.47.

### Examples for the pharmaceutical, compositions comprising the crystalline form produced by the method of the present invention

The examples of pharmaceutical formulation of the present invention are below. These examples are given in order to elucidate the subject of the present invention, though the subject of the present invention is not limited to these examples.

### Example 1: Dry powder inhalation formulation

| Content | Amount (mg) |
|---|---|
| Tiotropium bromide | 0.0220 |
| Lactose monohydrate (d₅₀=150µm) | 19.9500 |
| Lactose monohydrate (d₅₀=10µm) | 5.0280 |
| Total weight | 25 |

### Example 2: Dry powder inhalation formulation

| Content | Amount (mg) |
|---|---|
| Tiotropium bromide | 0.0215 |
| Lactose monohydrate (d50=150µm) | 19.9500 |
| Lactose monohydrate (d50=10µm) | 5.0285 |
| Total weight | 25 |

### Example 3: Pressurized metered-dose inhalation formulation

| Content | Amount (mg) |
|---|---|
| Tiotropium bromide | 0.0225 |
| HFA 134 | 74.98 |
| Total weight | 75.0025 |

### Example 4: Pressurized metered-dose inhalation formulation

| Content | Amount (mg) |
|---|---|
| Tiotropium bromide | 0.0217 |
| HFA 227 | 74.98 |
| Total weight | 75.0017 |

The present invention relates to the methods which are used in production of the crystalline form of tiotropium bromide.

The crystalline form of tiotropium bromide can be obtained by the method of crystallizing any crystalline form of Tiotropium bromide existing in the prior art with organic solvents and/or water.

The crystalline form of tiotropium bromide can be obtained by the method of crystallizing amorphous form of Tiotropium bromide with organic solvents and/or water.

The crystalline form of tiotropium bromide can be obtained by the method of transforming the crystalline form into amorphous form first and then crystallizing it with organic solvents and/or water.

The process of transforming any crystalline form of tiotropium bromide to amorphous form in the prior art can be carried out by generally known methods. However, the inventors have seen that the most successful results are obtained when tiotropium bromide in crystalline form is dissolved in water and the obtained solution is lyophilised.

The organic solvent or solvents that can be used for obtaining the crystalline form of the present invention are selected from a group comprising n-octane, cyclopentane, cyclohexane, n-butyl chloride, methyl t-butyl ether, xylene, chlorobenzene, o-diclorobenzene, ethylene dichloride, n-butyl alcohol, isopropyl alcohol, n-butyl acetate, isobutyl alcohol, methyl isoamyl ketone, n-propyl alcohol, chloroform, methyl isobutyl ketone, methyl n-propyl ketone, 1,4-dioxane, pyridine, 2-methoxyethanol, propylene carbonate, ethanol, dimethylformamide (DMF), dimethyl sulfoxide (DMSO), benzene, toluene, diethyl ether, tetrahydrofuran, methanol, acctonitrilc, acetone, ethyl acetate, methyl ethyl ketone, dichloromethane, dimethylacetamide, N-methyl pyrrolidone, hexane, heptane or a combination thereof.

In the invention, an organic solvent having a polarity index value in the range of 0.01 and 5.0, preferably in the range of 0.1 and 4.5 is used as the organic solvent. In another preferred embodiment of the present invention; cyclopentane, heptanes, hexane, n-octane, benzene, cyclohexane, n-butyl chloride, toluene, methyl t-butyl ether, o-xylene, chlorobenzene, o-diclorobenzene, ethyl ether, dichloromethane, ethylene dichloride, n-butyl alcohol, isopropyl alcohol, n-butyl acetate, isobutyl alcohol, methyl isoamyl alcohol, n-propyl alcohol, tetrahydrofuran, chloroform, methyl isobutyl ketone, ethyl acetate, methyl n-propyl ketone, methyl ethyl ketone or 1,4-dioxane is used. In a more preferred embodiment of the invention, n-octane or n-butyl acetate or an organic solvent composed of a combination thereof is used.

According to the present invention, there is a method that can be implemented in preparation of the crystalline form of tiotropium bromide, which is as follows;
I. Tiotropium bromide is dissolved in dichloromethane
II. The mixture obtained is mixed at a temperature between 1 and 15 °C
III. The solvent in the mixture is removed under low pressure
IV. The solid substance obtained is dried under low pressure
V. The solid substance dried is dissolved in water
VI. The solution obtained is lyophilised (it is dried under low pressure at a temperature in the range of 0 and -100 °C)
VII. The solid substance obtained as a result of lyophilisation is suspended by using solvents having a polarity index value in the range of 0.01 and 5.0
VIII. The suspension formed is heated up to a temperature in the range of 60-95°C
IX. The crystals precipitated are separated by filtration method
X. The crystals obtained are dried under low pressure.

In step V, 1 g tiotropium bromide is dissolved in approximately 20-100 ml water.

In step VII, the organic solvent to be used to suspend the solid substance obtained as a result of lyophilisation is selected among solvents having a polarity index value in the range of 0.01 and 5.0, preferably in the range of 0.1 and 4.5. In a preferred embodiment of the invention, n-butyl acetate or n-octane is used in this step.

The suspension obtained in step VIII is heated to a temperature in the range of 60 and 95°C in a preferred embodiment of the invention, a temperature in the range of 70 and 90°C in a more preferred embodiment of the invention.

The solid substance obtained as a result of implementation of step IV in the production method explained above comprises Tiotropium bromide (Formula I) and CH₂Cl₂ combination or a hydrate thereof, and the solid compound is characterized by having peaks at wavenumbers of 3109, 3074, 3057, 3023, 2979, 2970, 2955, 2935, 1747, 1479, 1433, 1348, 1324, 1244, 1161, 1071, 1039, 1000, 962, 886, 859, 797, 753, 665, 651, 626, 538, 432, 289, 253, 209 and 173 cm⁻¹ (stated as ± 2 cm⁻¹) in FT-Raman spectrum.

The term "compound comprising tiotropium bromide (Formula 1) and CH₂Cl₂ combination or a hydrate thereof' signifies that these two substances are in the same crystalline matrix or different crystalline/amorphous matrices.

The said compound comprising tiotropium bromide and CH₂Cl₂ combination is hereinafter referred as "tiotropium bromide DCM solvate".

### Examples related to preparation method of crystalline form of tiotropium bromide of the present invention:

The examples related to preparation method of the crystalline form of tiotropium bromide produced by the method of the present invention are given below. These examples arc given in order to elucidate the subject of the present invention, though the subject of the present invention is not limited to these examples.

### X-Ray Powder Diffraction

The measurements have been carried out with Bruker D8 Advance powder X-ray diffractometer which uses Cu Kα source of radiation in Bragg-Brentano reflection geometry. Generally, the 2θ values are accurate within an error of ±0.1-0.2°. Relative peak intensity can change significantly for different samples which have the same crystalline form due to different positioning of crystals. The samples have not been subjected to any special operation except a slight pressure applied in order to obtain a smooth surface. Single-crystal silicon sample holders having 1.0, 0.5 mm or 0.1 mm depth and 12 mm cavity diameter have been used. Tube voltage and current values are respectively 40 kV and 40 mA. The X-ray powder diffractometer is equipped with a LynxEye detector. A variable divergence slight is used with a 3 ° window. Step size is 0.02 °2θ, and step time is 37 seconds. The samples have been rotated at the rotation speed of 0.5 per second during measurement.

Structural resolution and refinement of crystalline structure is implemented with the help of direct methods (programmes such as SHELXS86, SHELXS97) and FMLQ refinement (programmes such as TeXsan, SMART etc.).

The pattern analysis implemented has shown that the crystalline anhydrous tiotropium bromide has a monoclinic cell having the following sizes:
a = 9.94 Å, b = 11.95 Å, c = 18.57 Å, β = 108.47°,

At the same time, the crystalline form of tiotropium bromide produced by the method of the present invention is characterized by peaks at values of 11.9, 13.6, 14.9, 16.8, 17.5, 18.7, 20.3, 21.5, 23.7 =0.2 2θ in X-ray diffraction. In another aspect, the crystalline form of tiotropium bromide produced by the method of the present invention is characterized by peaks at values of 14.1, 15.7, 18.3, 19.4, 19.8, 22.3, 24.6 ±0.2 2θ.

### FT-Raman spectroscopy

Raman spectrums have been recorded by Bruker RFS100 Raman spectrometer equipped with a germanium detector and Nd:YAG laser with an excitation wavelength of 1064 nm. A few milligrams of the substance have been pressed into aluminium sample holders. Spectra have been measured in the range of 50-3500 cm⁻¹ and with resolution of 2 cm⁻¹ by using laser power of 300 mW. 64 scans have been collected.

### Example 1:

Tiotropium bromide (10 g) is dissolved in 50 ml of dichloromethane. The mixture is mixed at room temperature for 24 hours. Then, dichloromethane comprised in the mixture is removed under low pressure. The solid substance obtained is dried under low pressure. The dried substance is dissolved in 100 ml of water. The obtained mixture is dried under low pressure at -80°C (is lyophilized). N-butyl acetate is added to the solid substance obtained and the solution is mixed at 100°C for 5 hours. The crystals obtained at the end of this time are separated from the mixture by filtration method. The obtained crystals are dried under low pressure.

## Claims

1. A method for producing anhydrous tiotropium bromide having monoclinic elementary cell parameters of a = 9.94 Å, b =11,95 Å, c = 18,57 Å, β = 108,47° determined by X-ray analysis, **characterized in** comprising steps of
I. Dissolving tiotropium bromide in dichloromethane,
II. Stirring the mixture at a temperature between 1 and 15 °C,
III. Removing the solvent in the mixture under low pressure,
IV. Drying the solid substance obtained under low pressure.
V. Dissolving the dried solid substance in water,
VI. Lyophilizing the obtained solution (dried under low pressure at a temperature in the range of 0 and -100 °C),
VII. Suspending the solid substance obtained as a result of lyophilisation by using solvents having a polarity index value in the range of 0.01 and 5.0,
VIII. Heating the suspension to a temperature in the range of 60 and 95 °C,
IX. Separating the crystals precipitated by filtration method,
X. Drying the obtained crystals under low pressure.

2. The method according to claim 1, **characterized in that** the organic solvent that shall be used to suspend the solid substance obtained at the end of lyophilisation is n-butyl acetate and/or n-octane.

## Patentansprüche

1. Verfahren zur Herstellung von wasserfreiem Tiotropiumbromid mit monoklinischen elementaren Zellparametern von a = 9.94 Å, b =11,95 Å, c = 18,57 Å, β = 108,47°, das durch X-Ray-Analyse bestimmt wird, **dadurch gekennzeichnet, dass** es die Schritte von
I. Lösen von Tiotropiumbromid in Dichlormethan,
II. Rühren der Mischung bei einer Temperatur zwischen 1 und 15°C,
III. Entfernen des Lösungsmittels im Gemisch unter niedrigem Druck,
IV. Trocknen der unter niedrigem Druck erhaltenen festen Substanz,
V. Lösen der getrockneten festen Substanz in Wasser,
VI. Lyophilisieren der erhaltenen Lösung (Trocknen unter niedrigem Druck bei einer Temperatur im Bereich von 0 bis -100°C),
VII. Suspendieren der durch Lyophilisation erhaltenen festen Substanz unter Verwendung von Lösungsmitteln mit einem Polaritätsindexwert im Bereich von 0.01 und 5.0,
VIII. Erwärmen der Suspension auf eine Temperatur im Bereich von 60 und 95°C,
IX. Trennung der durch das Filtrationsverfahren ausgefällten Kristalle,
X. Trocknen der erhaltenen Kristalle unter niedrigem Druck.

2. Verfahren nach Anspruch 1, **dadurch gekennzeichnet, dass** das organische Lösungsmittel, das zur Suspendierung der am Ende der Lyophilisation erhaltenen festen Substanz verwendet werden soll, n-Butylacetat und/oder n-Octan ist.

## Revendications

1. Procédé de production de bromure de tiotropium anhydre ayant des paramètres de cellules élémentaires monocliniques élémentaires de a = 9.94 Å, b =11,95 Å, c = 18,57 Å, β = 108,47° déterminée par l'analyse aux rayons X, **caractérisé en ce qu'**il comprend les étapes consistant à
I. Dissoudre le bromure de tiotropium dans le dichlorométhane,
II. Agiter le mélange à une température comprise entre 1 et 15°C,
III. Éliminer le solvant dans le mélange sous basse pression,
IV. Sécher la substance solide obtenue sous basse pression.
V. Dissoudre la substance solide séchée dans l'eau,
VI. Lyophiliser la solution obtenue (séchée sous basse pression à une température comprise entre 0 et -1 00°C),
VII. Suspendre la substance solide obtenue par lyophilisation en utilisant des solvants ayant un indice de polarité compris entre 0.01 et 5.0,
VIII. Chauffer la suspension à une température comprise entre 60 et 95°C,
IX. Séparer les cristaux précipités par filtration,
X. Sécher les cristaux obtenus sous basse pression.

2. Procédé selon la revendication 1, **caractérisé en ce que** le solvant organique qui doit être utilisé pour mettre en suspension la substance solide obtenue à la fin de la lyophilisation est l'acétate de n-butyle et/ou le n-octane.
